# FASCICULE DE BREVET EUROPEEN

(11) **EP 0 587 476 B1**
(45) Date de publication et mention de la délivrance du brevet: **18.03.1998**
(21) Numéro de dépôt: 93402135.3
(22) Date de dépôt: 01.09.1993
(51) Int. Cl.: A61K 31/195

(54) **Compositions renfermant des acide aminé mono ou polyhydroxylé pour le traitement du diabète non insulinodépendant**
Zusammensetzungen, die mono oder polyhydroxylierte Aminosäuren zur Behandlung des Insulinunabhängigen Diabetes Mellitus enthalten
Compositions containing mono or polyhydroxylated amino acids for the treatment of non-insulin dependent diabetes mellitus

(30) Priorité: 07.09.1992 FR 9210644
(43) Date de publication de la demande: 16.03.1994
(73) Titulaire: Société Civile JOUVENET, F-75016 Paris (FR)
(72) Inventeur: Sauvaire, Yves, F-34980 Montferrier sur Lez (Hérault) (FR); Ribes, Gérard, F-34070 Montpellier (FR)
(74) Mandataire: Cabinet HERRBURGER

(56) Documents cités:
- FR-A- 2 315 916
- PHYTOCHEMISTRY vol. 12, no. 7 , 1973 pages 1707 - 1711 FOWDEN, L. ET AL '4-HYDROXYISOLEUCINE FROM SEED OF TRIGONELLA FOENUM-GRAECUM'
- THE BRITISH JOURNAL OF NUTRITION vol. 68, no. 1 , Juillet 1992 pages 217 - 29 EVANS, A.J. ET AL 'RELATIONSHIP BETWEEN STRUCTURE AND FUNCTION OF DIETARY FIBRE: A COMPARATIVE STUDY OF THE EFFECTS OF THREE GALACTOMANNANS ON CHOLESTEROL METABOLISM IN THE RAT'
- LIPIDS vol. 26, no. 3 , Mars 1991 pages 191 - 197 SAUVAIRE, Y. ET AL 'IMPLICATION OF STEROID SAPONINS AND SAPOGENINS IN THE HYPOCHOLESTEROLEMIC EFFECT OF FENUGREEK'
- EUROPEAN JOURNAL OF CLINICAL NUTRITION vol. 42, no. 1 , Janvier 1988 pages 51 - 54 NADAR, Z. ET AL 'GLUCOSE-LOWERING EFFECT OF FENUGREEK IN NON-INSULIN DEPENDENT DIABETICS'
- PHYTOCHEMISTRY vol. 28, no. 7 , 1989 pages 1835 - 41 ALCOCK, N.W. ET AL 'STEREOCHEMISTRY OF THE 4-HYDROXYISOLEUCINE FROM TRIGONELLA FOENUM-GRAECUM'

## Description

La présente invention se rapporte à une composition antidiabétique spécialement destinée au traitement du diabète de type II ou diabète non insulino-dépendant.

Il est connu que le diabète touche, aujourd'hui, plus de trente millions d'individus dans le monde, prenant la dimension d'un phénomène majeur sur le plan de la santé publique : à titre d'exemple on considère que, dans les pays européens, le diabète atteint entre 2 et 5 % de la population, et qu'en France, environ 3 à 4 % des habitants souffrent de diabète non insulino-dépendant qui est de loin le plus fréquent et en particulier entre 5 et 10 % des sujets âgés de 60 à 70 ans souffrent de cette maladie.

De plus, et pour diverses raisons liées notamment à la richesse alimentaire, l'obésité, le tabagisme ou encore la diminution de l'activité physique, le nombre de diabétiques aurait doublé en France en une vingtaine d'années, essentiellement du fait d'une augmentation du diabète non insulino-dépendant.

Cette affection est caractérisée par un défaut de régulation de la sécrétion d'insuline associée ou non à une insulino-résistance des tissus périphériques. L'altération du fonctionnement des cellules B pancréatiques qui synthétisent l'insuline, survient dès la phase initiale du diabète non insulino-dépendant. Elle se traduit par une très nette diminution de la sécrétion d'insuline en réponse à une stimulation glucosée.

Pour traiter cette maladie, les spécialistes ont en conséquence été tout naturellement amenés à rechercher des produits susceptibles de stimuler la sécrétion d'insuline ; parmi ceux-ci, seuls les sulfamides (sulfonylurées) ont révélé une efficacité : ce sont, en conséquence, les seuls médicaments de ce type qui sont actuellement proposés sur le marché.

Cependant, malgré leurs avantages, les sulfonylurées présentent un certain nombre d'inconvénients avant tout liés aux difficultés rencontrées pour déterminer la posologie adéquate ; il en résulte des risques de surdosage pouvant provoquer fréquemment des hypoglycémies, avec risque de coma hypoglycémique, notamment chez les personnes âgées.

Il serait en conséquence souhaitable de pouvoir disposer d'un médicament de nature à se substituer aux sulfonylurées pour stimuler la sécrétion d'insuline, tout en ne présentant pas les inconvénients susmentionnés.

Il s'agit là du but que l'on s'est fixé conformément à l'invention.

Pour parvenir à celui-ci, on s'est souvenu que les anciens préconisaient, pour le traitement du diabète, des décoctions de graines d'une espèce particulière de trigonelle qui apparaissait déjà dans les pharmacopées grecques et latines : le fenugrec, Trigonella foenum graecum L., légumineuse facilement cultivable dans la région méditerranéenne.

On a alors eu l'idée de vérifier l'activité de cette plante, et d'analyser celle-ci par des fractionnements très poussés dans le but de rechercher d'éventuels constituants qui pourraient être responsables de cette activité.

On a ainsi été amené à mettre en lumière les propriétés antidiabétiques de certains dérivés d'acides aminés.

L'invention concerne donc une composition antidiabétique contenant de tels dérivés.

Cette composition antidiabétique est caractérisée en ce qu'elle renferme en tant que substance active, à l'état libre ou combiné, au moins un acide aminé mono ou polyhydroxylé préalablement synthétisé ou isolé et/ou ses formes lactoniques et ses dérivés amidés.

Selon une autre caractéristique de l'invention, la composition renferme un produit provenant de la métabolisation de la substance active.

Parmi les acides aminés susmentionnés, le plus actif s'est révélé être la 4-hydroxyisoleucine de formule : 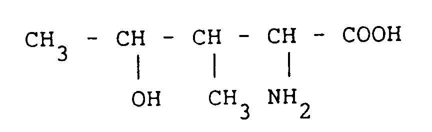 et/ou sa forme lactonique.

En conséquence, la composition antidiabétique conforme à l'invention renferme de préférence ce composé.

Cette composition peut être administrée par voie orale, intraveineuse ou intramusculaire, et renferme des excipients qui sont choisis en fonction de la forme galénique adoptée.

La posologie peut elle aussi varier dans de larges limites sans pour cela sortir du cadre de l'invention et dépend, en fait de chaque cas particulier à traiter.

La substance active peut, bien entendu, sans sortir du cadre de l'invention être d'origine quelconque, et en particulier être obtenue par synthèse. Cependant, et pour des raisons à la fois d'ordre philosophique et écologique, les spécialistes cherchent de plus en plus à proposer des produits dits "naturels" et la composition conforme à l'invention est avantageusement dérivée du règne végétal.

On a, à cet effet, pu constater que les trigonelles : Trigonella sp. renferment des quantités utilisables non négligeables d'acides aminés hydroxylés conformes à l'invention présentant une activité antidiabétique, et en particulier que les graines de fenugrec renferment en quantité notable la 4-hydroxyisoleucine.

L'invention se rapporte en particulier à une composition douée de propriétés insulino stimulantes pouvant servir de réactif pour l'exploration fonctionnelle du pancréas endocrine.

Un procédé d'obtention de la 4-hydroxyisoleucine à partir de graines de fenugrec sera décrit ci-dessous dans l'exemple 1 :

### Exemple 1

On rassemble des graines de fenugrec que l'on soumet à un broyage et à une extraction préalable à l'hexane à température ambiante de façon à obtenir 100 grammes de tourteau délipidé.

On soumet ensuite ce tourteau à six extractions hydro alcooliques successives avec de l'éthanol à 70 % à température ambiante (volume total : 2100 ml).

On concentre ensuite l'extrait obtenu sous pression réduite à 130 ml, et on fait passer le concentrat sur une résine échangeuse de cations sous forme H⁺ (AMBERLITE IR 120, ou DOWEX 50Wx8) dans une colonne de 36 cm de hauteur et 2 cm de diamètre de façon à retenir le produit recherché sur cette colonne. Celui-ci est ensuite élué en utilisant une solution d'ammoniaque N ou 2N.

Après concentration et reprise par de l'éthanol à 70 %, le mélange est soumis à une chromatographie d'adsorption sur une colonne de 50 cm de hauteur et de 2,5 cm de diamètre remplie de gel de silice 60, 70-230 mesh. On sépare ainsi la 4-hydroxyisoleucine qui est éluée avec de l'éthanol à 70 % (250 ml).

Le produit est ensuite concentré sous vide et purifié par cristallisation avec addition d'éther diéthylique.

On a ainsi pu obtenir 0,6 g de 4-hydroxyisoleucine de formule : 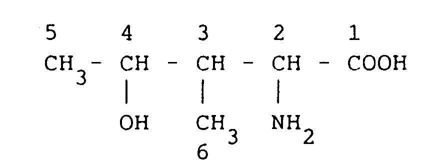 titrant 99 % de pureté qui a été identifiée et caractérisée de la manière indiquée ci-dessous :

### - Chromatographie sur couche mince (C.C.M)

Une solution hydro alcoolique (éthanol 70 % eau 30 %) de 4-hydroxyisoleucine ayant une concentration de 4 mg.ml⁻¹ est déposée sur une plaque recouverte de gel de silice G, soumise à une élution avec un mélange n-butanol/CH₃COOH/H₂O (3/2/1) ou phénol/eau (3:1). La plaque est ensuite chauffée à 110 C pendant 10 minutes et une solution acétonique de ninhydrine à 0,1 % est pulvérisée.

La 4-hydroxyisoleucine donne une seule tâche rouge-orangée à violette avec les deux systèmes de solvants. Les Rf sont respectivement les suivants :
Rf (n Butanol-CH₃COOH-H₂O) : 0,36
Rf (Phenol-H₂O) : 0,45

### - Spectroscopie R.M.N. (Appareil VARIAN EM 390)

Cette analyse a été effectuée à 90 MHz en utilisant une solution de 4-hydroxyisoleucine dans D₂O, et en tant que standard interne le T.S.S (Acide trimethyl silyl propane, sel de Na).

Les résonances ont été observées pour les valeurs suivantes :
- 0,95 et 1,25 ppm.
   Ces doublets sont attribuables respectivement aux protons des groupes CH₃ des carbones C₆ et C₅.
- 1,85 ppm
   Ce multiplet est attribuable au proton du C₃.
- 3,85 ppm
   Ce multiplet est composé d'un doublet dû au proton du carbone C₂ et d'un multiplet dû au proton du carbone C₄

### - Spectrométrie de masse par impact électronique

Cette analyse a été réalisée sur un appareil JEOL JMS D 100 à 75 eV.

Les fragmentations suivantes ont été obtenues :
148[M + H]⁺ : 5 % , 102 [M + H - CO₂H₂]⁺ : 32 %
74[M + H - 74]⁺ = 92 % , 58 [M + H - 74 - 16]⁺ : 100 %

### - Spectroscopie de masse par bombardement d'atomes rapides ou méthode FAB ("Fast Atom Bombardement").

Il s'agit là d'une technique récente qui comporte une ionisation plus douce. Elle est, de ce fait, beaucoup mieux adaptée à la caractérisation des composés polaires labiles.

La méthode FAB est basée sur le principe d'une production d'ions caractéristiques de la structure du composé testé par bombardement de celui-ci par un faisceau d'atomes rapides.

Le spectre de masse obtenu par cette méthode d'ionisation comporte :
- l'ion quasi moléculaire [M + H]⁺ présent à m/z 148 : 100 %
- la perte d'une molécule d'eau est mise en évidence par la fragmentation m/z : 130 : 35 %
- la perte de CO₂H₂ à partir de m/z 148, se traduit par la formation de l'ion m/z 102 : 30 %
- l'ion m/z : 74 : 56 % correspond à la rupture en deux fragments (ion et neutre) de l'ion [M + H]⁺

Afin d'établir que ces trois fragmentations se produisent bien à partir de l'ion m/z : 148 [M + H]⁺, celui-ci est soumis à une autre collision selon la technique FAB/MS/MS. La composition des ions formés correspond bien à la filiation mise en évidence précédemment.

Les analyses susmentionnées permettent clairement de mettre en évidence l'identité et la pureté de la 4-hydroxyisoleucine obtenue ; on a en conséquence pu utiliser ce composé pour la mise en oeuvre de tests pharmacologiques permettant de vérifier l'activité antidiabétique de la 4-hydroxyisoleucine ; ces tests qui ont été respectivement effectués in vitro au niveau des cellules et des organes et in vivo chez l'animal sont résumés dans les exemples 2, 3, 4 et 5.

Dans ces exemples, les hormones pancréatiques (insuline et glucagon) ont été évaluées par dosages radio-immunologiques.

La glycémie a été dosée à l'auto-analyseur Technicon par la méthode au ferricyanure de potassium.

Les résultats ont été soumis à une analyse de variance suivie du test de comparaison multiple.

### Exemple 2.

### Investigation au niveau des îlots de Langherans isolés de pancréas de rat.

Après digestion du pancréas par la collagénase, les îlots de Langerhans qui possèdent des cellules B sécrétant l'insuline ont été séparés des autres éléments du digestat, prélevés sous la loupe binoculaire, puis déposés dans des tubes d'incubation. Cette méthode, qui présente l'avantage de ne nécessiter qu'une faible quantité de substance permet une étude directe des effets de la composition sur des cellules endocrines pancréatiques, en particulier des cellules B insulino-sécrétrices, à l'exclusion de toute interférence avec les tissus exocrines et annexes.

Sur des îlots isolés de rats Wistar, incubés en présence de 8,3 mM de glucose (1,5 g/l) pendant une heure, on a recherché l'effet de différentes concentrations de 4-hydroxyisoleucine sur la sécrétion d'insuline. Les résultats obtenus sont reportés sur le schéma joint en annexe 1 sur lequel les histogrammes représentent pour chaque dose de 4-hydroxyisoleucine la sécrétion d'insuline mesurée sur 60 minutes.

Sur ce schéma, on peut noter qu'un effet stimulant sur la sécrétion apparaît pour une concentration de 4-hydroxyisoleucine de 200 µM (avec une incertitude inférieure à 5 %). Cette stimulation de la sécrétion augmente légèrement en fonction de la dose de 4-hydroxyisoleucine.

A titre comparatif, on a observé dans les mêmes conditions les effets de deux analogues structuraux de la 4-hydroxyisoleucine : l'isoleucine et la leucine, et on a pu constater que pour ces deux substances, l'effet stimulant sur la sécrétion d'insuline n'apparaît que pour des concentrations 50 à 100 fois supérieures.

### Exemple 3

### Investigation au niveau du pancréas isolé et perfusé de rat.

Pour réaliser ce test, le pancréas a été totalement isolé de tous les autres organes et tissus voisins (rate, estomac, duodénum, graisse épiloïque) et perfusé en circuit ouvert dans une chambre de perfusion, avec une solution physiologique. Cette préparation présente l'avantage de respecter l'intégrité fonctionnelle et vasculaire de l'organe, tout en le soustrayant aux influences régulatrices (humorales ou nerveuses) auxquelles il est habituellement soumis.

Dans cet exemple, les expériences ont été réalisées en présence d'une concentration de glucose de 8,3 mM. Dans ces conditions, on a étudié l'effet sur la sécrétion d'insuline d'une perfusion de 4-hydroxyisoleucine à la concentration de 200 µM pendant 10 minutes. Le schéma joint en annexe 2, sur lequel les résultats de cette expérimentation sont reportés montrent clairement que la stimulation est immédiate et biphasique et persiste pendant toute la durée de la perfusion.

Pour "affiner" ces résultats, on a également étudié l'effet sur la sécrétion d'insuline de la 4-hydroxyisoleucine à des concentrations de 50 et 500 µM. Les quantités d'insuline sécrétées pendant les 10 minutes de perfusion, sont indiquées ci-dessous :

| | Sécrétion d'insuline (ng/10 mn) |
|---|---|
| Glucose seul | 810 ± 83 |
| Glucose + 4-hydroxyisoleucine (50 µM) | 1232 ± 93 |
| Glucose + 4-hydroxyisoleucine (200 µM) | 1520 ± 154 |
| Glucose + 4-hydroxyisoleucine (500 µM) | 2206 ± 213 |

Ce tableau montre que l'effet stimulant augmente en fonction de la concentration de 4-hydroxyisoleucine.

Il est à noter qu'au cours de ces expériences, on n'a observé aucune modification du débit vasculaire pancréatique ni du taux de glucagon pancréatique, hormone de contrerégulation qui tend in vivo à augmenter le taux de glycémie et par conséquent à atténuer les effets de la stimulation de la sécrétion d'insuline.

Les résultats montrent clairement que la stimulation de la sécrétion d'insuline observée en présence de la composition est due à une stimulation directe de la cellule B de l'ilôt de Langherans.

### Exemple 4

### Expérimentation "in vivo" chez le rat

Au cours de cette expérimentation, des rats Wistar ont été anesthésiés puis munis de cathéters dans les deux veines jugulaires. Un cathéter permet le prélèvement d'échantillons de sang nécessaires au dosage du glucose et de l'insuline plasmatiques. L'autre cathéter est utilisé pour l'injection intraveineuse de la substance à tester.

Le tableau joint en annexe 3 montre, chez le rat Wistar anesthésié, préalablement nourri ad libitum, l'effet d'une administration aiguë par voie intraveineuse de 4-hydroxyisoleucine à la dose de 9 mg/kg de poids vif sur l'insuline plasmatique d'une part, et la glycémie d'autre part.

Ce tableau montre que l'administration de 4-hydroxyisoleucine déclenche une augmentation immédiate et particulièrement importante du taux d'insuline plasmatique. La conséquence de cette hyperinsulinémie est une diminution de la glycémie. Cette réduction du taux de glucose circulant devient significative 15 minutes après l'injection et atteint environ 30 % par rapport à la valeur de départ à la 90ème minute.

Dans ces conditions expérimentales, aucun effet latéral secondaire à l'injection de 4-hydroxyisoleucine n'a été observé : ni polypnée, ni tachycardie, ni hypoxie.

Toujours chez le rat normal anesthésié, préalablement nourri ad libitum, un test de tolérance au glucose par voie intraveineuse a été réalisé.

Le glucose dissous a été administré à la dose de 0,3 g/kg par voie intraveineuse, dans une des deux veines jugulaires du rat. Le schéma joint à l'annexe 4 montre que la glycémie s'élève immédiatement, culmine dès la 3^{ème} minute, à en moyenne 282 mg/dl (soit +112 %). Lorsque la 4-hydroxyisoleucine (9 mg/kg) est injectée par voie intraveineuse en même temps que le glucose, l'hyperglycémie provoquée est nettement réduite, la glycémie n'atteint à la 3^{ème} minute que 204 mg/dl (soit +58 %).

### Exemple 5

### Expérimentation "in vivo" chez le chien

Au cours d'une expérimentation complémentaire chez le chien, nous avons recherché si la 4-hydroxyisoleucine peut être active après administration par voie orale et si, dans ce cas, cette substance conserve sa propriété insulino-sécrétrice et par conséquent sa faculté d'améliorer la tolérance au glucose.

Chez des chiens normaux éveillés maintenus à jeun depuis 18 heures, la 4-hydroxyisoleucine est dissoute dans du sérum physiologique et elle est administrée par intubation gastrique à la dose de 9 mg/kg.

Des prélèvements sanguins effectués dans une des deux veines jugulaires ont permis d'évaluer l'insulinémie et la glycémie de ces animaux. Le schéma de l'annexe 5 montre que la 4-hydroxyisoleucine déclenche une élévation très rapide et importante de l'insulinémie qui atteint +277 % dès la 3^{ème} minute. Cette hyperinsulinémie , qui dans nos conditions de jeûne ne dure que 5 minutes, est accompagnée d'une réduction progressive et discrète de la glycémie.

Une épreuve d'hyperglycémie provoquée par voie orale a été ensuite réalisée dans les mêmes conditions chez le chien.

Le glucose dissous a été administré par intubation gastrique à la dose de 1 g/kg.

### Exemple 6

La toxicité aiguë a été réalisée chez la souris SWISS des deux sexes lors de l'administration par voie intrapéritonéale d'une dose unique de 1 gramme par kilo de 4-hydroxyisoleucine. L'observation de ces animaux pendant une période de 15 jours suivant l'administration ne semble mettre en évidence aucun phénomène toxique. La D.L. 0 (dose léthale nulle) est dans nos conditions expérimentales supérieure à 1 gramme par kilo.

L'autopsie réalisée au terme de cette étude ne semble révéler aucune altération macroscopique sur les principaux organes observés : tymus, coeur, poumons, foie, estomac, reins, surénals, pancréas, duodénum, tractus génital et vessie.

Le schéma de l'annexe 6 montre que la glycémie, dosée sur du sang prélevé dans une veine jugulaire, s'élève progressivement dès la 5^{ème} minute et atteint son maximum à la 30^{ème} minute (166 mg/dl, soit +85 %). Lorsque la 4-hydroxyisoleucine (9 mg/kg) est ajoutée à la solution glucosée administrée par intubation gastrique, la glycémie n'atteint que 120 mg/dl à la 30ème minute, soit uniquement +31 %.

Les tests susmentionnés montrent clairement que la 4-hydroxyisoleucine est une substance qui a la propriété de stimuler puissamment la sécrétion d'insuline à tous les niveaux d'organisation : cellulaire, organe isolé, animal entier. "In vivo" l'induction de l'hyperinsulinémie a pour conséquence une réduction de la glycémie que la 4-hydroxyisoleucine soit administrée par voie intraveineuse ou par voie orale. Les tests "in vitro" démontrent que la 4-hydroxyisoleucine, aux concentrations de l'ordre de la µM, stimule la sécrétion d'insuline par un effet direct sur la cellule B de l'îlot de Langerhans.

Il est donc clair que la 4-hydroxyisoleucine pourrait avantageusement être utilisée en tant que produit actif dans des compositions destinées à la thérapeutique du diabète non insulino-dépendant aussi bien par voie orale que par voie intraveineuse. Cette possibilité est renforcée par le fait que la 4-hydroxyisoleucine améliore la tolérance au glucose par voie intraveineuse ou par voie orale dans les deux espèces animales étudiées : rat et chien.

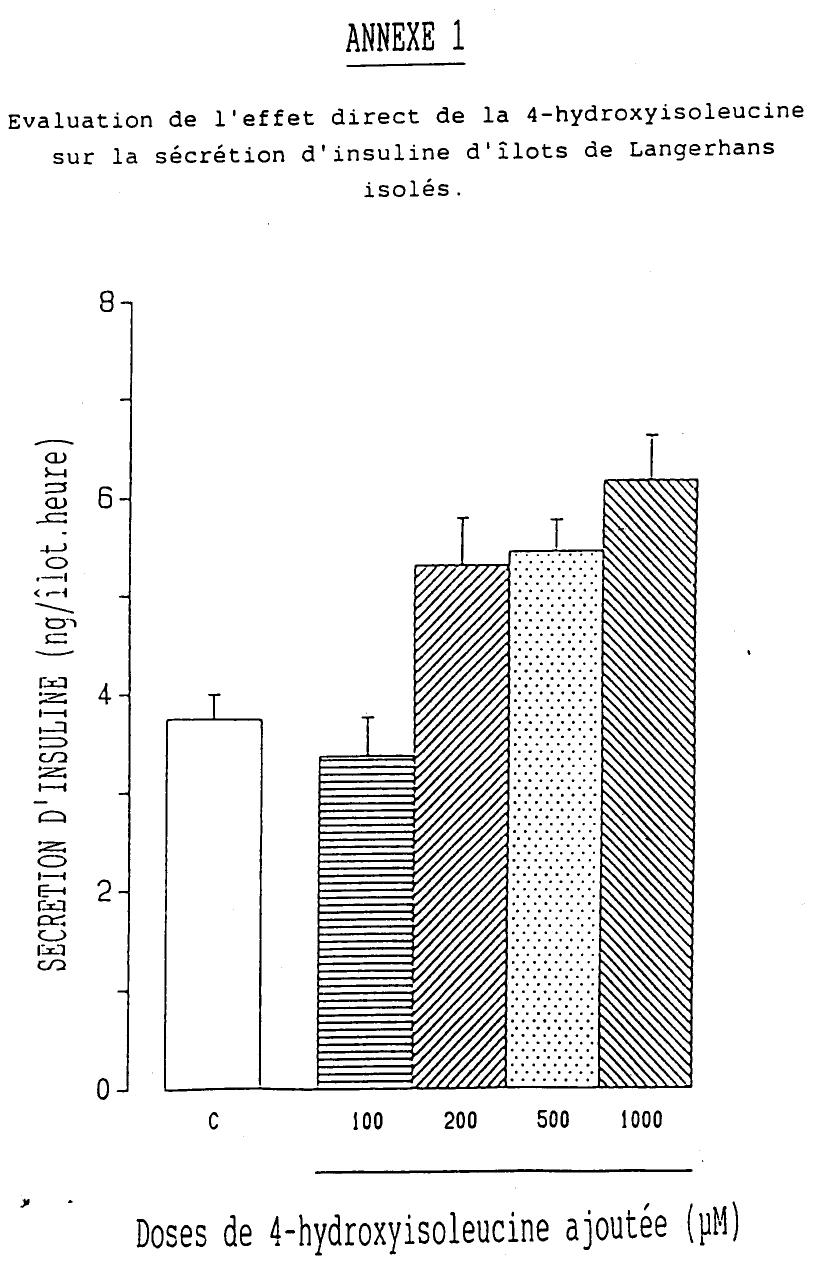

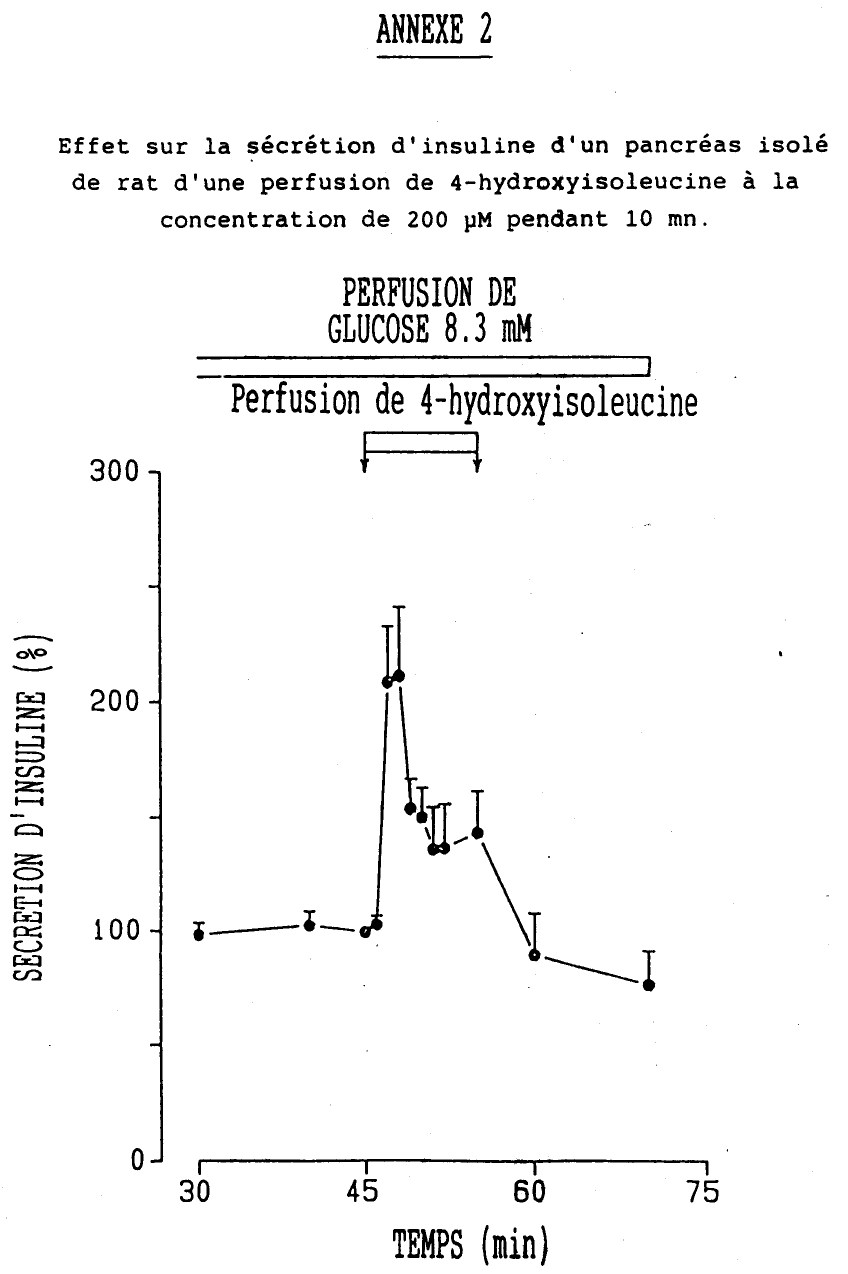

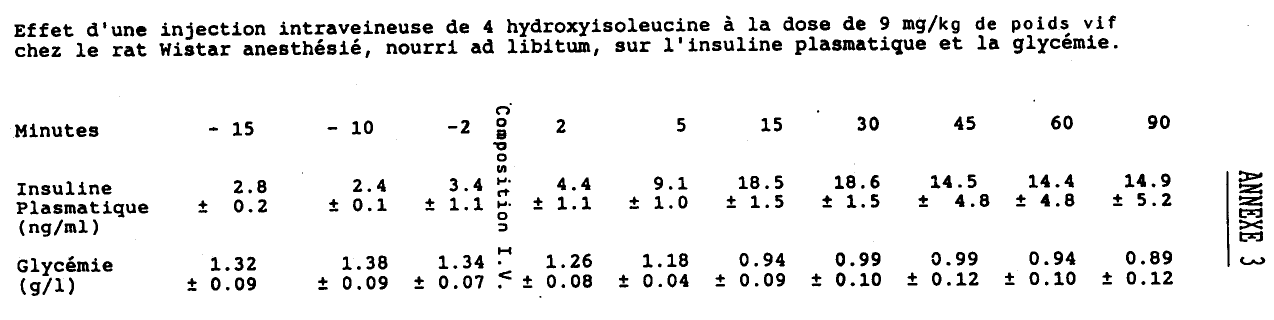

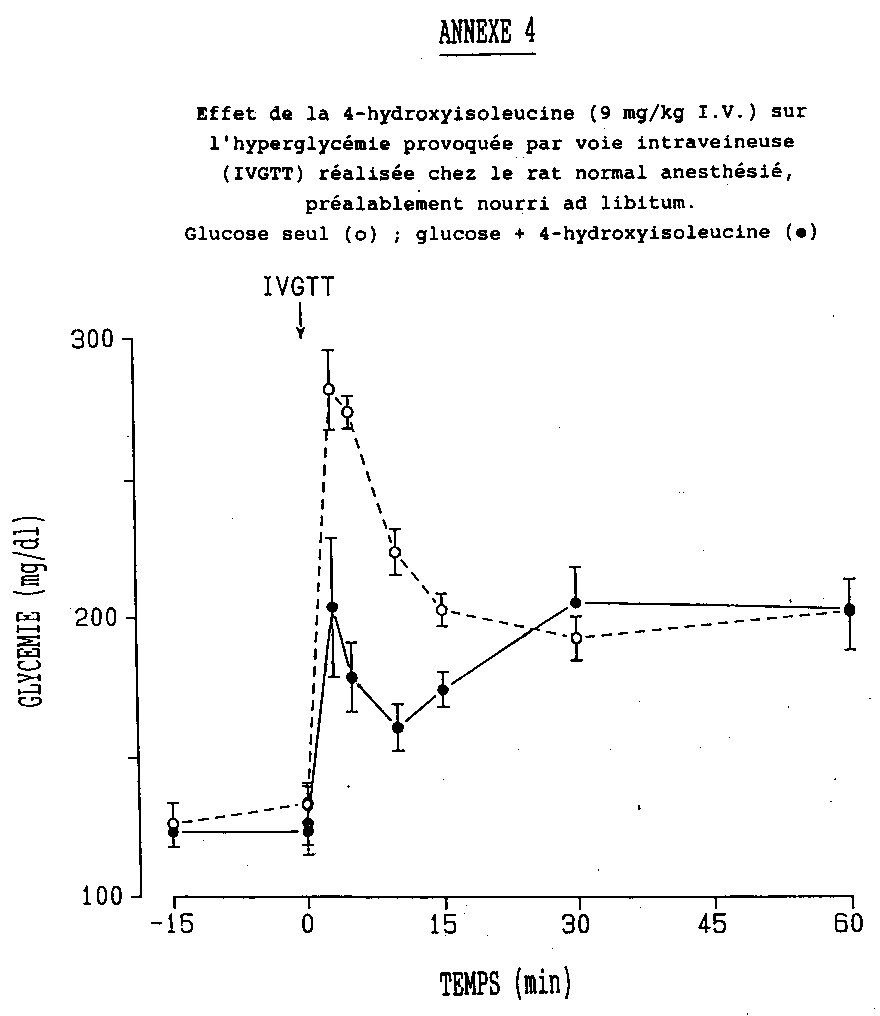

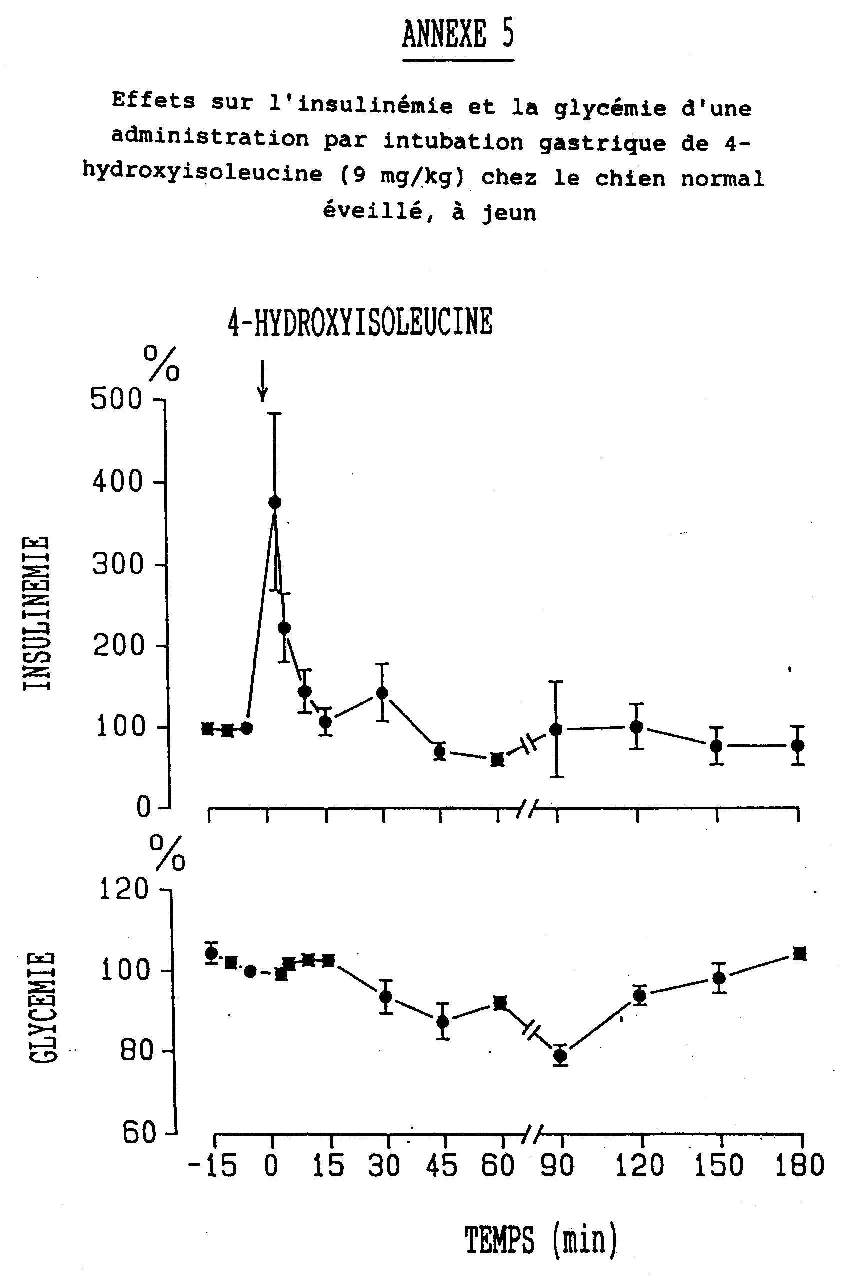

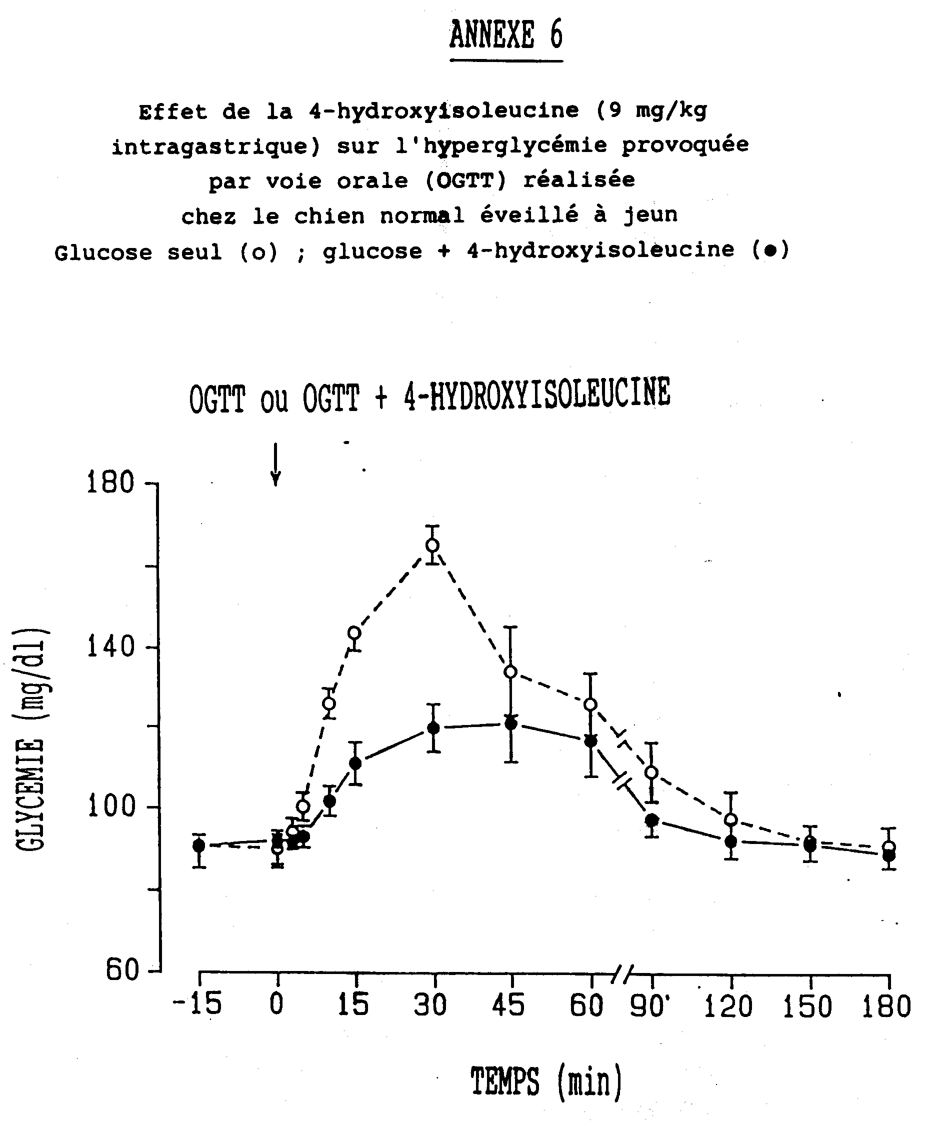

## Revendications

1. Composition renfermant, en tant que substance active à l'état libre ou combiné, au moins un acide aminé mono- ou polyhydroxylé préalablement synthétisé ou isolé et/ou ses formes lactoniques et ses dérivés amidés pour utilisation comme médicament.

2. Composition selon la revendication 1, caractérisée en ce qu'elle renferme un produit provenant de la métabolisation de la substance active.

3. Composition selon l'une quelconque des revendications 1 et 2, caractérisée en ce que la substance active est la 4-hydroxyisoleucine de formule :

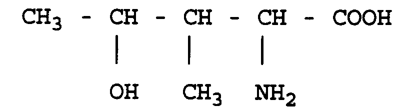

4. Composition selon l'une quelconque des revendications 1 à 3, caractérisée en ce que la substance active est d'origine végétale.

5. Composition selon la revendication 4, caractérisée en ce que la substance active est obtenue à partir de trigonelles (Trigonella sp.).

6. Composition selon la revendication 5, caractérisée en ce que la substance active est extraite de graines de fenugrec (Trigonella foenum graecum L).

7. Composition selon l'une quelconque des revendications 1 à 6, douée de propriétés isulino stimulantes, caractérisée en ce qu'elle peut servir de réactif pour l'exploration fonctionnelle du pancréas endrocine.

8. Utilisation de la composition selon l'une quelconque des revendications 1 à 7, pour l'obtention d'un médicament destiné au traitement du diabète non isulino dépendant.

## Claims

1. Composition containing, as active substance in a free or combined state, at least ono mono- or polyhydroxylated amino acid synthesized or isolated beforehand and/or its lactone forms and its amide derivatives, for use as a medicament.

2. Composition according to Claim 1, characterized in that it contains a product derived from the metabolism of the active substance.

3. Composition according to either of Claims 1 or 2, characterized in that the active substance is 4-hydroxyisoleucine of formula:

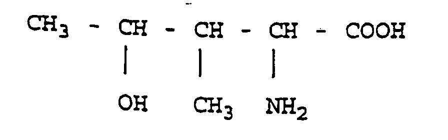

4. Composition according to any one of Claims 1 to 3, characterized in that the active substance is of plant origin.

5. Composition according to Claim 4, characterized tin that the active substance is obtained from trigonella (Trigonella sp.).

6. Composition according to Claim 5, characterized in that the active substance is extracted from fenugreek seeds (Trigonella foenum graecum L).

7. Composition according to any one of Claims 1 to 6, endowed with insulin-stimulating properties, characterized in that it can serve as reagent for the functional exploration of the endocrine pancreas.

8. Use of a composition according to any one of Claims 1 to 7, for the production of a medicament intended for the treatment of non-insulin-dependent diabetes.

## Patentansprüche

1. Zusammensetzung, die mindestens eine vorher hergestellte oder isolierte mono- oder polyhydroxylierte Aminosäure und/oder ihre Lactonformen und Amidderivate als Wirkstoff - in freier oder kombinierter Form - zur Verwendung als Arzneimittel enthält.

2. Zusammensetzung nach Anspruch 1, dadurch gekennzeichnet, daß sie ein aus der Metabolisierung des Wirkstoffs stammmendes Produkt enthält.

3. Zusammensetzung nach einem der Ansprüche 1 und 2, dadurch gekennzeichnet, daß es sich bei dem Wirkstoff um 4-Hydroxyisoleucin der Formel 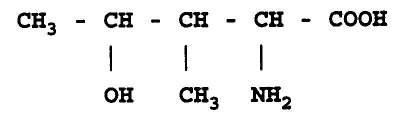 handelt.

4. Zusammensetzung nach einem der Ansprüche 1 bis 3, dadurch gekennzeichnet, daß der Wirkstoff pflanzlichen Ursprungs ist.

5. Zusammensetzung nach Anspruch 4, dadurch gekennzeichnet, daß der Wirkstoff aus Boxhornklee (Trigonella sp.) stammt.

6. Zusammensetzung nach Anspruch 5, dadurch gekennzeichnet, daß der Wirkstoff aus Boxhornkleesamen (Trigonella foenum graecum L) gewonnen wird.

7. Zusammensetzung nach einem der Ansprüche 1 bis 6 mit insulinstimulierenden Eigenschaften, dadurch gekennzeichnet, daß sie als Reagens für die Untersuchung der endokrinen Pankreasfunktion dienen kann.

8. Verwendung der Zusammensetzung nach einem der Ansprüche 1 bis 7 zur Herstellung eines Arzneimittels zur Behandlung der insulinunabhängigen Diabetes.
